# EUROPEAN PATENT APPLICATION

(11) **EP 1 964 586 A1**
(43) Date of publication of application: **03.09.2008**
(21) Application number: 08356031.8
(22) Date of filing: 03.03.2008
(51) Int. Cl.: A61M 5/32

(54) **Shield for covering the extremity of an administration device or assembly, an assembly and an administration device**

(30) Priority: 02.03.2007 FR 0701531
(71) Applicant: Becton Dickinson France, 38800 Le Pont-de-Claix (FR)
(72) Inventor: Felix-Faure, Catherine, 38000 Grenoble (FR)
(74) Representative: Brédeville, Odile Marie

(57) **Abstract**

The present invention relates to a shield (1) for covering at least part of a needle (2) and needle hub (6) of a needle assembly, said shield (1) having an open proximal end (1a), a closed distal end (1b) and a longitudinal wall (4) extending from said proximal end (1a) to said distal end (1b) and defining an interior cavity (5) for receiving at least part of the needle (2) and needle hub (6), characterized in that it comprises a first bulge (8) defined on said wall (4) and extending into said interior cavity (5), said first bulge (8) being designed to be able to, resiliently and releasably, engage a part of said needle assembly to secure said shield (3) thereto, said first bulge (8) further being circumferentially non-continuous about said interior cavity (5).

The invention also relates to a needle assembly and to an injection device, each comprising such a shield.

## Description

The present invention relates to a shield for covering the extremity of an administration device or of an assembly at least prior to use of the administration device or of the assembly.

In this application, the term distal means the part furthest from the user's hand, and the term proximal means the part closest to the user's hand. Likewise, in this application, the term "distal direction" means the direction of administration, i.e., towards the patient, and the term "proximal direction" means direction opposite to the direction of administration, i.e., away from the patient.

Aministration devices are commonly used in several technical fields such as, for example, the medical field, to administer to a patient, for example a medical product, eit her by spraying for spraying devices or by injection for injection devices. To do so, the extremity of the administration device can be provided with a staked needle or nozzle or a luer connection allowing provision of an assembly such as a nozzle assembly or a needle assembly.

In the present application, by "staked needle" one means a needle fixed on the tip of the injection device by gluing or by any other suitable method such as for example shrinking of the tip of the syringe surrounding the needle after heating.

In the medical field, injection devices, such as syringes provided with needles, either staked needles or needle assemblies on luer syringes, are usually provided to the end-users with needle shields: indeed, needles must remain sterilized until use and need to be protected from possible contamination from the environment. Similarly, injection device such as syringe with no needle, provided luer or with nozzle or with luer lock fitting, are usually provided to the end-users with a cap protecting the extremity of the luer or of the nozzle from possible contamination from the environment. Moreover, in the case of injection devices with needle, the end user must also be protected from accidental needle-stick injuries.

These needle shields or caps are usually made of an elastic material, such as a thermoplastic elastomer, and are assembled on the syringe extremity for instance on assembly lines of industrial pharmaceutical companies. In the case of syringe with staked needle, the distal end of the syringe usually comprises a bulging part or hub to which the needle is secured by gluing and upon which the needle shield is removably engaged by friction, for example. One of the problems encountered during such an assembly step of the needle shield on the hub is the generation or the revealing of particles caused by the friction between the needle shield and the bulging part of the syringe hub that bears the needle. This causes important quality issues. This problem may also occur when the needle shield is withdrawn from the syringe hub.

In this application, the term "revealing" means that some particles may already exist or be preformed in the needle shield and are displaced and/or broken away during the assembling or removing of the needle shield from the syringe hub.

Similar problems may occur with syringes provided luer or with nozzles covered by caps.

Moreover, similar problems may occur with assemblies such as nozzle assembly or needle assembly provided to be connected to a luer spraying or injection device.

In addition to the previous problem described, once the syringe or any other administration device is delivered to the end-user, the needle shield must be removed from the syringe hub in order to free the needle before the syringe is ready for an injection. This operation may be delicate, since the end user must take care not to remove the needle shield too quickly in order to avoid aspiration, by the needle shield and through the needle, of the liquid product contained in the syringe. At the same time, because the needle shield must sealingly close the syringe hub for reasons of sterility, the needle shield is tightly fixed to the syringe hub and its removal may be quite difficult. As previously mentioned, similar problem may occur with other administration devices provided luer or with spraying or injection assemblies.

US 6,719,732 B2 discloses a protection device for a syringe needle comprising an elastic needle shield of general longitudinal direction presenting a closed distal end and an open proximal end, the needle shield being formed by a lateral wall defining an inner housing intended to receive the hub of a syringe body. A generally continuous annular bead is provided on the lateral wall and disposed in the housing and includes at least one slot extending longitudinally through the annular bead. The slot permits sterilizing gas to pass into the housing without causing the needle shield to detach from the syringe during the sterilization process. The annular bead provides for generally continuous contact between the needle shield and the syringe - except for the slot. Therefore, the needle shield disclosed in this patent is held in place on the syringe in a securely way. Nevertheless, the assembling of the needle shield on the syringe and its withdrawal require to exert forces that can be felt to be too important by the users. In addition, theses forces may lead to particules generation or revealing linked with the high level of friction between the annular bead and the syringe.

There is therefore a need for a protection device that could be securely fixed to an administration device or to an assembly so as to safely and efficiently protect the extremity of the administration device or of the assembly and would be, at the same time:
- on a process level easy to assemble on the tip of an administration device such as a syringe hub or on an assembly, and
- on an end-user level easy to disassemble,
and in both cases that would limit the generation or revealing of particles. The present invention overcomes the above-described shortcomings of the prior art.

In accordance with an embodiment of the present invention, there is proposed a shield for covering at least part of the extremity of an administration device or of an assembly, said shield having an open proximal end, a closed distal end and a longitudinal wall extending from said proximal end to said distal end and defining an interior cavity for receiving at least part of the extremity of the administration device or of the assembly, characterized in that it comprises a first bulge defined on said wall and extending into said interior cavity, said first bulge being designed to be able to, resiliently and releasably, engage a part of said extremity of the administration device or of the assembly to secure said shield thereto, said first bulge further being circumferentially non-continuous about said interior cavity.

The fact that the shield does not have any annular bead permits to decrease the forces required to assemble the shield to the administration device or to the assembly, or to withdraw it. In addition, the limited contact area between the shield and the administration device or assembly decreases the particles generation and revealing.

Preferably, the shield of the invention comprises at least one second bulge having at least its shape or one of its dimensions different from said first bulge, said second bulge further being circumferentially non-continuous about said interior cavity and resiliently and releasably engaging the same part of said extremity of the administration device or assembly (20) as said first bulge.

In an embodiment of the invention, the shield comprises at least two first or second bulges, both being located in the same transversal plane.

In another embodiment of the invention, the shield comprises at least two first or second bulges, both being located in different transversal planes.

Preferably, said first and/or second bulges are regularly spaced from each other.

In an embodiment of the invention, at least one of said first or second bulge has a proximal face forming a predetermined angle β with the longitudinal axis of said shield. Preferably, said predetermined angle β ranges from 35° to 60°.

In an embodiment of the invention, at least one of said first or second bulge has a distal face forming a predetermined angle α with the longitudinal axis of said shield. Preferably, said predetermined angle α ranges from 20° to 40°.

Because of the specific angular arrangement of the proximal face of the bulge, the removal of the shield from the extremity of an administration device or from an assembly by the end user is facilitated. In particular, the angular arrangement creates a smooth slope that allows the end user to retrieve the shield slowly without having to pull too greatly on the shield: the risk of aspiration of liquid and/or of accidental needle stick injury due to a too sharp movement is therefore prevented. In addition the slope formed by the specific angular arrangement of the distal face of the bulge facilitates the assembly of the shield on the extremity of an administration device or of an assembly before it is provided to the end user, hence reducing particles generation and/or revealing.

In an embodiment of the invention, at least part of said shield is made from an elastically deformable material and is contained within a shell made at least in part from a rigid material. The shield is therefore protected from external shocks and more easily handled for assembling and removal steps. In addition, when the administration device is an injection device provided with a needle, or when the assembly comprises a needle, the rigid material prevents the needle from piercing the shield when, after use of the injection device, the user puts the shield back over the needle.

In an embodiment of the invention, at least one of said first or second bulge have the cross-sectional shape of half a drop of water of which widest part faces the distal end of said shield. This specific shape improves the reliability of the positioning and the holding of the shield on the administration device or on the assembly and enables the removal of the shield with a reduced force.

In an embodiment of the invention, said first and/or second bulges define for said cavity a predetermined geometric shape. Said predetermined geometric shape may be chosen in the group comprising a square, a triangle, an oblong format, a cross, a star.

Advantageously, the shield further comprises an outer casing formed of a first material and an inner casing formed of a second material different from the said first material, said inner casing defining said interior cavity for receiving, in an impermeable way, at least part of the extremity of the administration device or of the assembly, and aspiration limiting means for limiting the deformation of said interior cavity when said shield is separated from the assembly.

The aspiration limiting means may comprise bearing surfaces designed for being in contact with said inner casing and for cooperating with said inner casing in order to limit the stretching of said inner casing when said shield is separated from said administration device or assembly.

Another aspect of the invention is an assembly for an administration device, said assembly comprising at least an assembly hub for assembling said assembly on said administration device, characterized in that it further comprises a shield as described hereinabove.

Another aspect of the invention is an administration device comprising at least a container, and an extremity, characterized in that it further comprises a shield as described hereinabove provided on said extremity.

Other advantages of the present invention will now be specified with the aid of the description which follows and of the attached drawings in which:
Figure 1 is a cross section view of an embodiment of the shield of the invention,
Figure 2 is a perspective view of the shield of figure 1,
Figure 3 is a cross section view of the shield of figure 1 following the transversal plane P1,
Figures 4A to 4D are cross section views similar to figure 3 for alternative embodiments of the shield according to the invention,
Figure 5 is a cross section view of the shield of figure 1 with a syringe hub ready to be assembled on the shield,
Figure 6 is a cross section view of the shield of figure 5 once assembled on the syringe hub,
Figures 7A and 7B are cross section views of other alternative embodiments of the shield according to the invention,
Figure 8 is a cross section view of another alternative embodiment of the shield according to the invention contained in a shell,
Figures 9A to 9H are cross section views similar to figure 3 for alternative embodiments of the shield according to the invention,
Figure 10A is a partial cross section view of an alternative embodiment of the shield according to the invention of figure 7A, and
Figure 10B is a partial cross section view of an alternative embodiment of the shield according to the invention of figure 7B.

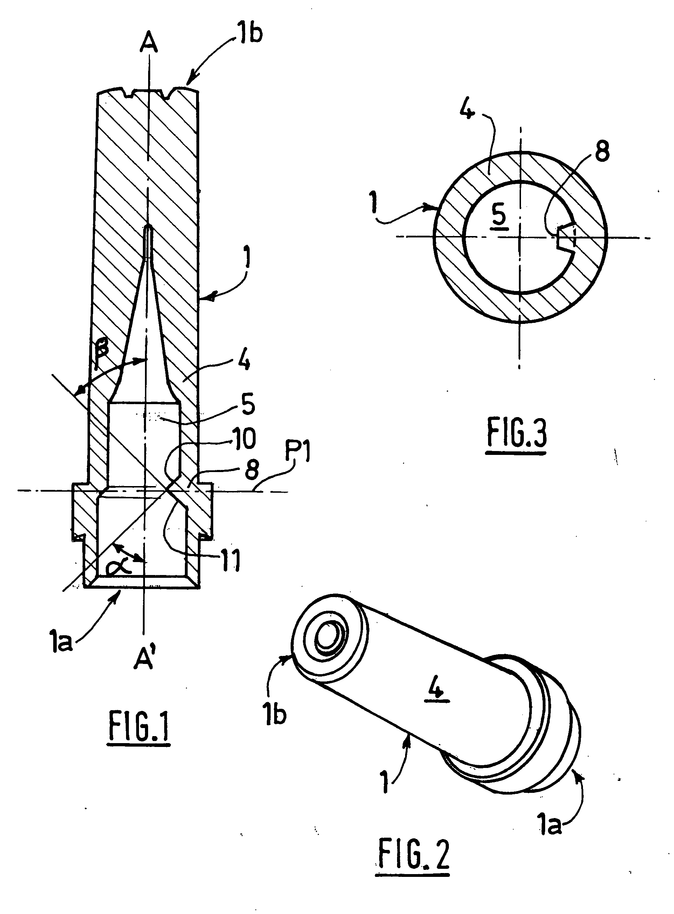

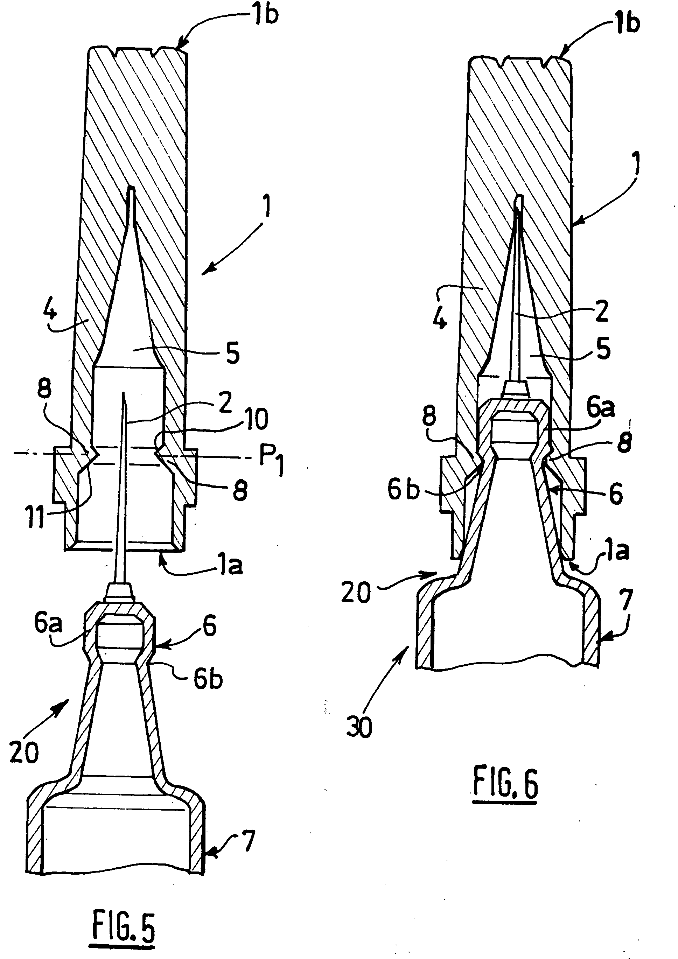

On Figures 1, 2 and 3 is shown a shield 1 according to the invention having the general shape of cap, with an open proximal end 1a, a closed distal end 1b and a longitudinal wall 4 extending from said proximal end 1 a to said distal end 1b and defining a cavity 5. This cavity 5 is intended to receive at least part of a needle 2 and needle hub 6 (both shown on figures 5 and 6). The shield 1 of figure 1 comprises a first bulge 8, defined on the internal face of the wall 4, said first bulge 8 extending into the interior cavity 5. As appears more clearly from figure 3, which is a cross section view of the shield 1 of figure 1 along the transversal plane P1, the first bulge 8 forms a circumferentially non-continuous projection inside the cavity 5.

As shown on figure 1, the first bulge 8 has a distal face 10 that forms an angle α with the longitudinal axis AA' of the shield 1, and a proximal face 11 that forms an angle β with said longitudinal axis AA'. The angle α preferably ranges from 20 to 40°. On the example shown, the angle α has a value of 30°. The angle β preferably ranges from 35 to 60°. On the example shown, the angle β has a value of 45°.

The shield 1 of figure 1 is made of an elastically deformable material, for instance a thermoplastic elastomer. The first bulge 8 is also made of an elastically deformable material, which may be identical or different from the material forming the shield. The first bulge 8 of the shield 1 of figure 1 is made of thermoplastic elastomer. The first bulge 8 is intended to engage resiliently and releasably a part of the extremity of an administration device 20 or of an assembly such as the needle hub 6 described in figure 5.

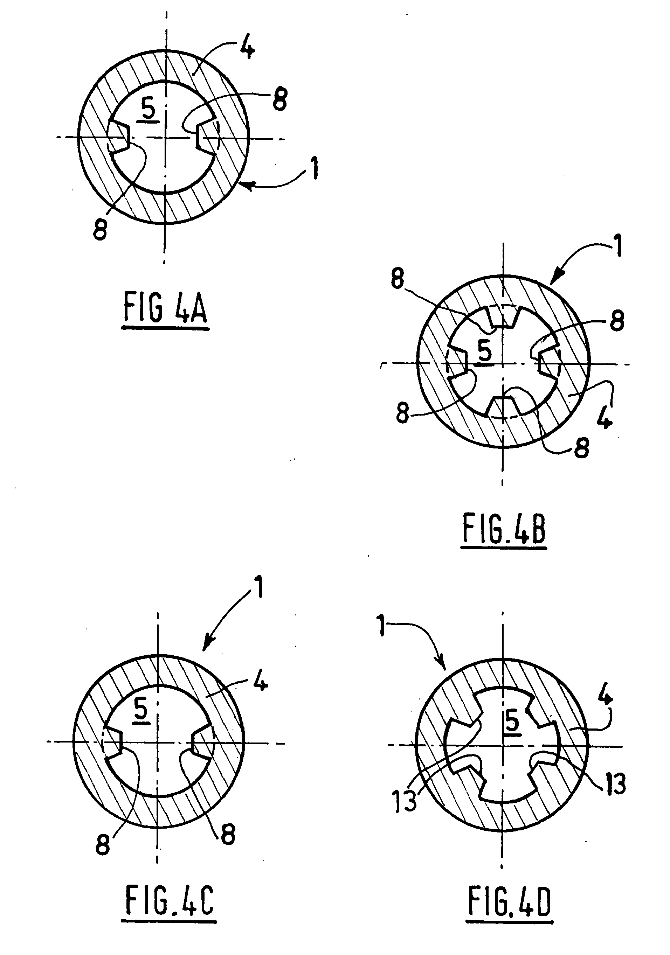

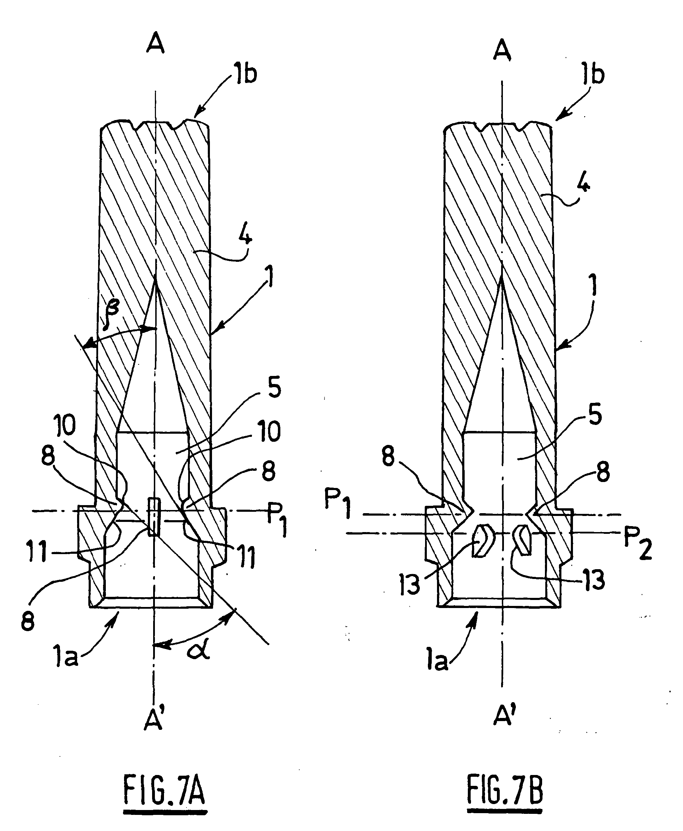

In alternate embodiments of the invention, the shield 1 comprises one or more additional bulges or second bulges, in addition to the first bulge 8 as described in reference with figure 1. The additional/second bulges may be identical or different in shape or in dimension from said first bulge 8. They may be located in the same transversal plane as the first bulge 8 or on the contrary in different transversal planes. The additional/second bulges form circumferentially non-continuous projections. Whatever the arrangement between the first bulge 8 and the additional/second bulges, the first bulge 8 and the additional/second bulges altogether form circumferentially non-continuous projections. Figures 7B, 4C, 4D are illustrative views, similar to figures 1, 3, of such alternate embodiments comprising additional/second bulges. In embodiments of the invention, additional/second bulges engage the same part, for instance a needle hub 6 as described in figure 5, of the extremity of the administration device 20 as the first bulge 8.

As will appear now from the description of figures 5 and 6, the first bulges 8 are designed to be able to, resiliently and releasably, engage a part of a needle assembly 20 to secure said shield 1 thereto.

On figure 5 is shown a variant of the shield 1 of figure 1 comprising two identical first bulges 8 defined on the internal face of the wall 4, said first bulges 8 extending into the interior cavity 5. The references designating the same elements as in figure 1 have been maintained on figures 5 and 6. As appears more clearly from figure 4A, which is a cross section view of the shield 1 of figure 5 along the transversal plane P1, the first bulges 8 are diametrically opposed one to the other and form circumferentially non-continuous projections. On figure 5 is also shown a needle hub 6 located at the distal end of a container 7 partially shown. The needle hub 6 comprises a distal end forming a bulging part 6a, which is separated from the container 7 by a portion of reduced diameter 6b of the needle hub 6. A needle 2 is fixed at the distal end of the bulging part 6a. As appears on figure 5, the diameter of the bulging part 6a is slightly larger than the diameter of the cavity 5 in the transversal plane P1.

In consequence, during the process of the shield 1 assembly on the needle hub 6, the first bulges 8 deform under the pressure exerted by the bulging part 6a. Once the shield 1 is in place, as shown on figure 6, the first bulges 8 come back in their rest state and are in contact with the portion of reduced diameter 6b of the needle hub 6. The shield 1 is therefore securely fixed to the needle hub 6.

Because the first bulges 8 form a circumferentially non-continuous projection, the assembly of the shield 1 does not require an excessive force and the installation of the shield 1 is facilitated. Also, it does not cause the generation of particles. In particular, because the proximal face 11 of the bulges 8 form an angle β of 45° with the longitudinal axis AA' of the shield 1 the assembly of the shield 1 on the needle hub 6 is smooth and eased. For the same reason, in particular because the first bulges 8 form a circumferentially non-continuous projection, the removal of the shield 1 from the needle hub 6 by the user is facilitated and does not require exerting an excessive pulling force. In addition, the fact that the distal face 10 of the first bulges 8 form an angle α of 30° with the longitudinal axis AA' of the shield 1 facilitates the removal of the shield 1 from the needle hub 6. When the user removes the shield 1 from the needle hub 6, the first bulges 8 deform under the stress exerted by the bulging part 6a and then return to their normal state thanks to their inherent resilience. Again, thanks to this angular arrangement, the generation or revealing of particles at the time of insertion and/or removal of the shield 1 is very limited.

On figure 7A is shown another alternative embodiment of the invention wherein the shield 1 comprises four identical first bulges 8 (among which only three are visible on the figure), defined on the internal face of the wall 4, said first bulges 8 extending into the interior cavity 5. The references designating the same elements as in figure 1 have been maintained on figure 7A. As appears more clearly from figure 4B, which is a cross section view of the shield 1 of figure 7A along the transversal plane P1, the first bulges 8 are located in the same transversal plane P1, regularly spaced and having the shape of half a drop of water. The widest part of the half drop of water faces the distal end 1b of the shield 1. Because of this specific shape of the first bulges 8, the distal face 10 of the first bulges 8 form an angle α of approximately 30° with the longitudinal axis of the shield 1, and the proximal face 11 of the first bulges 8 form an angle β of approximately 45° with the longitudinal axis of the shield 1. The installation and the removal of the shield 1 on a needle hub (not shown) are therefore facilitated.

On figure 7B is shown a further alternative embodiment of the shield 1 of figure 1 which comprises two first bulges 8 and four second bulges 13 defined on the internal face of the wall 4, said first and second bulges 8, 13 extending into the interior cavity 5. The first and second bulges 8, 13 are designed to be able to, resiliently and releasably, engage the same part, for instance a needle hub 6 such as described on figure 5, of a needle assembly 20 to secure said shield 1 thereto. The references designating the same elements as in figure 1 have been maintained on figure 7B. As appears more clearly from figure 4C, which is a cross section view of the shield 1 of figure 7B along the transversal plane P1, the first bulges 8 are located in a first transversal plane P1 and diametrically opposed one to the other and form circumferentially non-continuous projections. As appears more clearly from figure 4D, which is a cross section view of the shield 1 of figure 7B along the transversal plane P2, the second bulges 13 are located in the same second transversal plane P2 and regularly spaced. Such an arrangement of first and second bulges 8, 13, on different transversal planes P1, P2 allows to better maintain the shield 1 on the needle assembly 20 with a more uniformed maintaining force distributed on a larger portion of the needle hub 6 and without increasing the force required for the shield 1 removal.

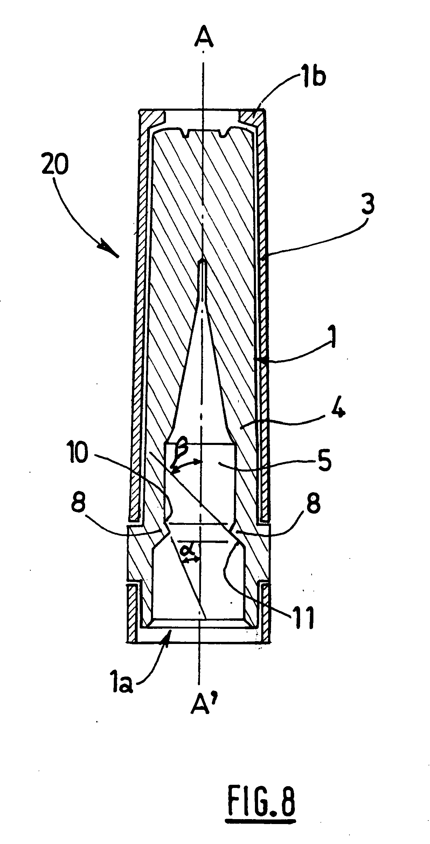

On figure 8 is shown the shield 1 of figure 5 contained in a shell 3. The shell 3 is made of a rigid material, such as polypropylene. The shell 3 is intended to protect the shield 1 from external shocks, to ease the shield 1 handling and prevent the needle (not shown) from piercing the shield 1.

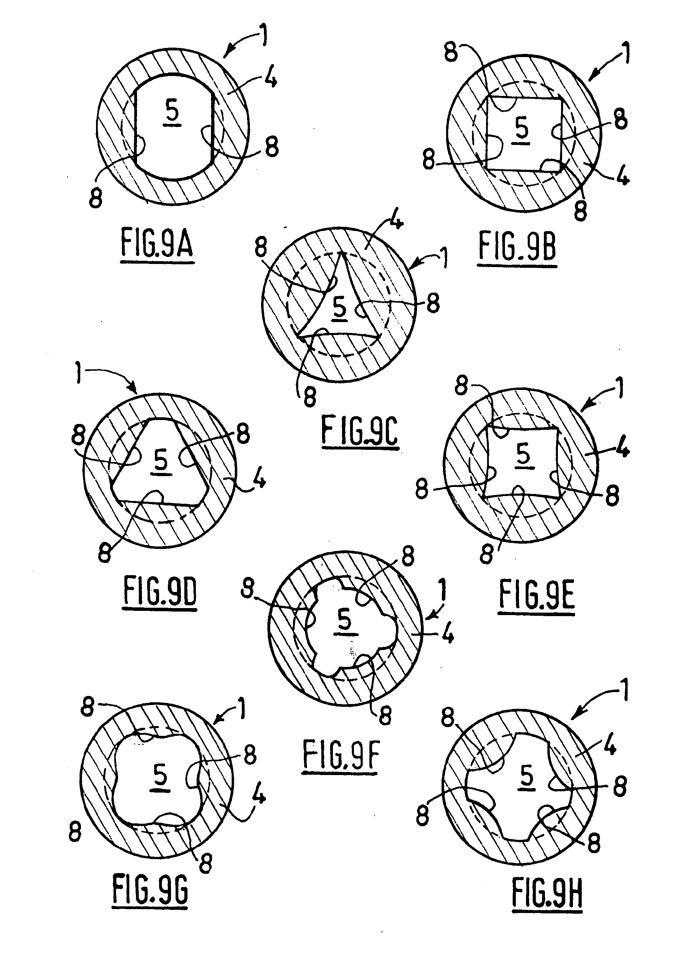

On figures 9A to 9H are shown cross section views similar to figure 3 for alternative embodiments of the shield 1 of the invention where the first bulges 8 define predetermined geometric shape. In particular, the first bulges 8 define for the cavity 5:
- in figure 9A, a shape of an oblong format,
- in figures 9B, 9E, and 9G, a shape similar to or substantially similar to a square,
- in figures 9C and 9D, a shape substantially similar to a triangle,
- in figure 9F, a shape substantially similar to a star,
- in figure 9H, a shape substantially similar to a cross.

The second bulges 13 can also define one of the predetermined geometric shapes described above.

In all figures 9A to 9H, the bulges 8 form a circumferentially non-continuous projection. The installation and/or removal of the shield 1 on the needle hub 6 are therefore facilitated.

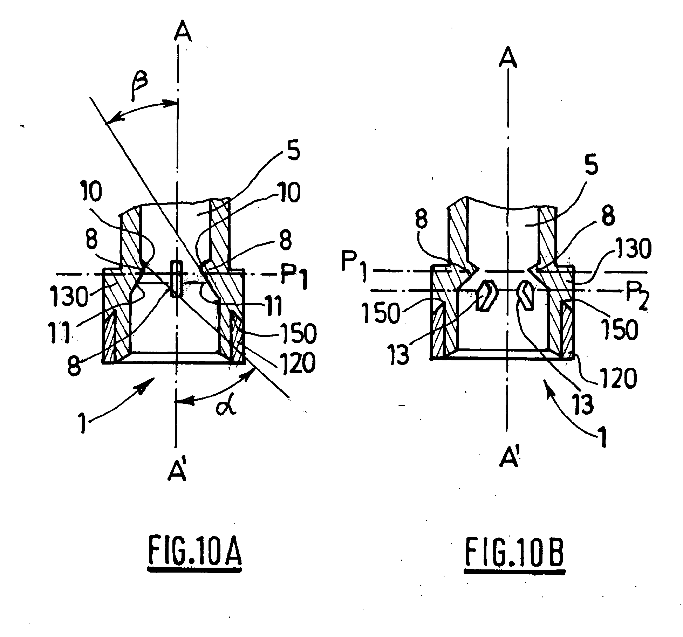

Figure 10A is a cross section view of an alternative embodiment of the shield 1 of figure 7A further comprising an outer casing 120 formed of a first material, and an inner casing 130 formed of a second material different from the said first material. The said first material and the said second material may show a different stiffness. The references designating the same elements as in figure 7A have been maintained in figure 10A. In the example shown on figure 10A, the inner casing 130 defines the interior cavity 5. The shield 1 of figure 10A further comprises a hook 150 formed on the outer casing 120. The hook 150 forms a bearing surface in contact with the inner casing 130. When the shield 1 is removed from the administration device or assembly (not shown), the hook 150 cooperates with the inner casing 130 so as to limit the stretching of the inner casing, in particular in regards to the outer casing 120 which is formed of a material different from the material forming the inner casing 130.

Indeed, when the shield 1 is removed from the administration device or assembly, a negative pressure may be created in the interior cavity 5, said negative pressure being likely to generate within said interior cavity the aspiration of the substance contained in the administration device or assembly. By limiting the stretching of the inner casing 130, the hook 150 forms an aspiration limiting means of the such created aspiration.

Figure 10B is a cross section view of an alternative embodiment of the shield 1 of figure 7B further comprising an outer casing 120 formed of a first material, and an inner casing 130 formed of a second material different from the said first material. The said first material and the said second material may show a different stiffness. The references designating the same elements as in figure 7B have been maintained in figure 10B.

Like the shield 1 of figure 10A, the shield 1 of figure 10B comprises a hook 150 formed on the outre casing 120, and constituting an aspiration limiting means of the aspiration created when the shield 1 is removed from the administration device or assembly (not shown).

The shield of the invention is an improvement of the needle shields of the prior art. Thanks to the shield of the invention, it is possible to efficiently protect a needle with a shield that may be assembled and/or removed on a syringe hub or needle assembly in a facilitated way and without generation or revealing of particles. Similarly, the shield of the invention can be efficiently used to protect the extremity of any kind of administration devices such as for example spraying devices and/or any kind of administration assemblies such as, for example, needle assembly, nozzle assembly.

## Claims

1. A shield (1) for covering at least part of the extremity of an administration device or of an assembly (20), said shield (1) having an open proximal end (1a), a closed distal end (1b) and a longitudinal wall (4) extending from said proximal end (1a) to said distal end (1b) and defining an interior cavity (5) for receiving at least part of the extremity of the administration device (2) or of the assembly (20), **characterized in that** it comprises a first bulge (8; 13) defined on said wall (4) and extending into said interior cavity (5), said first bulge (8; 13) being designed to be able to, resiliently and releasably, engage a part of said extremity of the administration device (2) or of the assembly (20) to secure said shield (1) thereto, said first bulge (8; 13) further being circumferentially non-continuous about said interior cavity (5).

2. A shield (1) according to claim 1, **characterized in that** it comprises at least one second bulge (13) having at least its shape or one of its dimension different from said first bulge (8), said second bulge (13) further being circumferentially non-continuous about said interior cavity and resiliently and releasably engaging the same part of said extremity of the administration device or assembly (20) as said first bulge (8).

3. A shield (1) according to claim 1 or 2, **characterized in that** it comprises at least two first or second bulges (8), both being located in the same transversal plane.

4. A shield (1) according to claim 1 or 2, **characterized in that** it comprises at least two first or second bulges (13), both being located in different transversal planes.

5. A shield (1) according to any of claims 2 to 4, **characterized in that** said first and/or second bulges (8, 13) are regularly spaced from each other.

6. A shield (1) according to claim 1 or 2, **characterized in that** at least one of said first or second bulge (8, 13) has a proximal face (11) forming a predetermined angle β with the longitudinal axis of said shield (1).

7. A shield (1) according to claim 6, **characterized in that** said predetermined angle β ranges from 35° to 60°.

8. A shield (1) according to claim 1 or 2, **characterized in that** at least one of said first or second bulge (8, 13) has a distal face (10) forming a predetermined angle α with the longitudinal axis of said shield (1).

9. A shield (1) according to claim 8, **characterized in that** said predetermined angle α ranges from 20° to 40°.

10. A shield (1) according to claim 1, **characterized in that** at least part of said shield (1) is made from an elastically deformable material and is contained within a shell (3) made at least in part from a rigid material.

11. A shield (1) according to claim 1 or 2, **characterized in that** at least one of said first or second bulge (8, 13) have the cross-sectional shape of half a drop of water of which widest part faces the distal end (1b) of said shield (1).

12. A shield (1) according to claim 3, **characterized in that** said first and/or second bulges (8, 13) define for said cavity (5) a predetermined geometric shape.

13. A shield (1) according to claim 12, **characterized in that** said predetermined geometric shape is chosen in the group comprising a square, a triangle, an oblong format, a cross, a star.

14. A shield (1) according to claim 1, **characterized in that** it further comprises:
an outer casing (120) formed of a first material;
an inner casing (130) formed of a second material different from the said first material, said inner casing (130) defining said interior cavity (5) for receiving, in an impermeable way, at least part of the extremity of the administration device or of the assembly, and
aspiration limiting means (150) for limiting the deformation of said interior cavity (5) when said shield (1) is separated from the assembly (20).

15. A shield (1) according to claim 14, **characterized in that** said aspiration limiting means comprise bearing surfaces (150) designed for being in contact with said inner casing (130) and for cooperating with said inner casing (130) in order to limit the stretching of said inner casing (130) when said shield (1) is separated from the assembly (20).

16. An assembly (20) for an administration device (30), said assembly (20) comprising at least an assembly hub (6) for assembling said assembly on said administration device, **characterized in that** it further comprises a shield (1) according to at least claim 1.

17. An administration device (30) comprising at least a container (7) and an extremity, **characterized in that** it further comprises a shield (1) according to at least claim 1 provided on said extremity.
